(19) Europäisches Patentamt
European Patent Office
Office européen des brevets

(11) Publication number: **0 369 014 B1**

(12) # EUROPEAN PATENT SPECIFICATION

(45) Date of publication of patent specification: **15.12.93**

(51) Int. Cl.⁵: **B01D 71/26**, B01D 71/30, B01D 71/56, A61M 1/16, A61M 1/34

(21) Application number: **88907382.1**

(22) Date of filing: **05.08.88**

(86) International application number: **PCT/JP88/00779**

(87) International publication number: **WO 89/00878 (09.02.89 89/04)**

(54) ## HYDROPHILIC POROUS MEMBRANE, PROCESS FOR ITS PRODUCTION AND PLASMA-SEPARATING APPARATUS.

(30) Priority: **06.08.87 JP 195381/87**

(43) Date of publication of application: **23.05.90 Bulletin 90/21**

(45) Publication of the grant of the patent: **15.12.93 Bulletin 93/50**

(84) Designated Contracting States: **BE DE FR GB IT NL SE**

(56) References cited:

| | |
|---|---|
| EP-A- 0 249 513 | FR-A- 2 080 241 |
| JP-A- 6 168 104 | JP-A- 6 327 540 |
| JP-A-51 140 885 | JP-A-58 163 405 |
| JP-A-59 160 504 | JP-A-62 179 540 |
| JP-A-62 262 705 | JP-A-63 145 662 |
| JP-B- 6 121 673 | |

(73) Proprietor: **TERUMO KABUSHIKI KAISHA**
**No. 44-1, Hatagaya 2-chome,**
**Shibuya-ku**
**Tokyo 151(JP)**

(72) Inventor: **ONISHI, Makoto Terumo Kabushiki Kaisha**
**2656-1, Ohbuchi**
**Fuji-shi Shizuoka 417(JP)**
Inventor: **FUJII, Tatsuya Terumo Kabushiki Kaisha**
**2656-1, Ohbuchi**
**Fuji-shi Shizuoka 417(JP)**

(74) Representative: **Gillard, Marie-Louise et al**
**Cabinet Beau de Loménie**
**158, rue de l'Université**
**F-75340 Paris Cédex 07 (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 369 014 B1

## Description

### Technical Field

This invention relates to a hydrophilic porous membrane, a method for the production thereof and a blood plasma separator. More particularly, it relates to a novel hydrophilic porous membrane suitable for blood plasma separating membrane exhibiting a high recovery ratio for the coagulation factor in the blood,possessing a low capacity for activation of the complement system, and excelling in blood compatibility and a method for the production thereof and a blood plasma separator.

### Background Art

The blood plasma separating membrane adapted to effect separation of blood into the blood cell component and the blood plasma component has been heretofore used for the blood plasma purification aimed at the elimination of abnormal proteins, immune complexes, antibodies, etc. from patients suffering from diseases due to abnormal immunity such as lupus erythematosus and arthrorheumatism and for the collection of blood plasma to be used for transfusion,for example. As blood plasma separating membranes of this nature, cellulose acetate membrane (Japanese Patent Laid-Open SHO 54(1979)-15,476), polyvinyl alcohol membrane, polyester membrane, polysulfone membrane, polyethylene membrane, and polypropylene membrane have been developed. In these membranes, the cellulose acetate membrane and the polyvinyl alcohol membrane exhibit affinity for water. It has been known that when these membranes are used for the separation of blood plasma, they inevitably entail activation of thecomplementary component in the blood plasma and the separated blood plasma is consequently obtained in a seriously injured state ("Jinko Zoki (Jpn. J. Artif. Organs)," Vol. 16, No. 2, pp. 1045 to 1050) (1987).

The hydrophobic blood plasma separating membranes of polypropylene and polyethylene exhibit no such high capacity for the activation of the complementary component as the hydrophilic membranes mentioned above. They, however, are liable to adsorb blood plasma proteins such as fiblinogens and consequently suffer from the disadvantage that they are not merely clogged readily but also deprived of their ability to recover such useful blood plasma proteins as fibrinogens and the factors VIII. Further, these hydrophobic membranes have a serious drawback that they must undergo a treatment for impartation of hydrophilicity before they are put to use. The proteins adsorbed on the membrane surface and consequently denatured, therefore, exert serious effects upon the blood plasma component or the blood cell component. In the circumstances, the desirability of developing a blood plasma separating membrane excellent in compatibility to blood has found recognition.

As concerns the compatibility of the material for membrane itself to blood, block copolymers which possess a micro-phase separation textures, grafted materials, etc. are being studied in search of feasibility. From the practical point of view, however, the technique for effective manufacture of blood plasma separating membranes from these materials remains yet to be established. The membranes so far trially manufactured with these materials have not fulfilled the expected functions to any appreciable extent.

Hydrophilic porous membranes used as blood plasma separating membranes have also been studied. EP-A-249 513 describes a hydrophilic porous membrane having graft chains of a hydrophilic monomer formed on the surface of a hydrophobic porous membrane. Said membrane exhibits dimensional stability and strength.

An object of this invention, therefore, is to provide a novel hydrophilic porous membrane and a method for the production thereof.

Another object of this invention is to provide a novel hydrophilic porous membrane suitable for hydrophilic blood plasma separating membrane exhibiting a high recovery ratio for coagulation factors in the blood possessing a low capacity for activation of the complement system, and excelling in compatibility to blood and to a method for the production thereof.

A further object of this invention is to provide a blood plasma separator which exhibits a high recovery ratio for coagulation factors in the blood possessing a low capacity for activation of the complement system, and excels in compatibility to blood.

### Disclosure of Invention

The objects of the invention described above are accomplished by a hydrophilic porous membrane comprising

a) a hydrophobic porous membrane having as a matrix thereof a hydrophobic polymer, $(X)_m$, wherein m is a degree of polymerization of the hydrophobic polymer composed of at least one kind of monomer units X of formula :

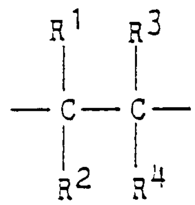

wherein $R^1$ and $R^2$ are independently H or F, $R^3$ is H, Cl, or F, and $R^4$ is H, Cl, F, or $CH_3$, providing that $R^2$ and $R^3$ are each H unless $R^1$, $R^2$, $R^3$ and $R^4$ are each invariably F and that $R^3$ is H unless $R^3$ and $R^4$ are each equally Cl or F, said monomer units being capable of forming a hydrophobic polymer; and

b) a hydrophilic polymer, $(Y)_n$, wherein n is a degree of polymerization of the hydrophilic polymer, composed of at least one kind of monomer units Y of formula :

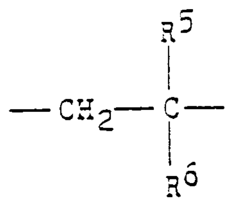

wherein $R^5$ is H or $CH_3$ and $R^6$ is

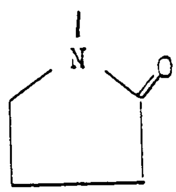 ,

$CONH_2$, $CONHR^7$,

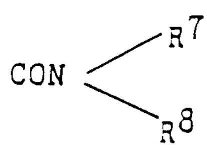

wherein $R^7$ and $R^8$ are independently an alkyl of 1 to 4 carbon atoms,

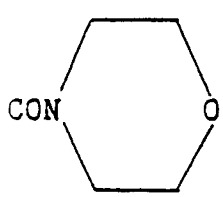 ,

COOM, wherein M is a metallic element, $COOR^9NHR^{10}$, wherein $R^9$ is an alkylene of 1 to 4 carbon atoms and $R^{10}$ is an alkyl of 1 to 4 carbon atoms,

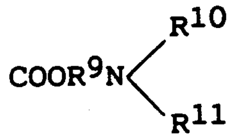,

wherein $R^9$ and $R^{10}$ have the same meanings as defined above and $R^{11}$ is an alkyl of 1 to 4 carbon atoms, or

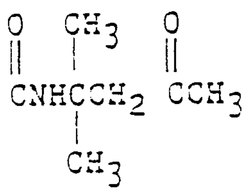,

providing that $R^5$ is H when $R^6$ is

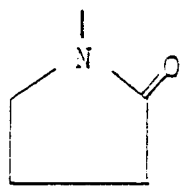,

said monomer units Y being capable of forming a hydrophilic monomer and graft polymerized to at least the surface of said porous membrane ; which hydrophilic porous membrane has the monomer units Y and the monomer units X present on the membrane surface at a molar ratio, Y/X, in the range of 0.2 to 2.0 and the component polymers present in the whole web of the membrane at a weight ratio $\{Y\}_n/\{X\}_m$, in the range of 0.03 to 0.3.

This invention also discloses a hydrophilic porous membrane whose hydrophobic polymer possesses a bubble point in the range of 0.1 to 5.0 kg/cm$^2$ and a wall thickness in the range of 50 to 500 microns and permeates water in an amount of not less than 5 ml/cm$^2$ min. under a pressure of 0.71 kg/cm$^2$. This invention further discloses a hydrophobic porous membrane whose component polymers are such that the subscript m has a value in the range of $10^2$ to $10^5$ and the subscript n a value in the range of 10 to $10^4$ in the respective formulas thereof. This invention discloses a hydrophilic porous membrane whose hydrophobic polymer is polypropylene, ethylenepropylene copolymer, or polyvinylidene fluoride. Further, this invention discloses a hydrophilic porous membrane whose monomer units Y are represented by the formula:

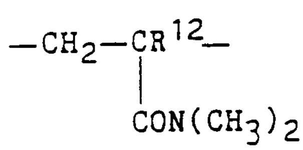

wherein $R^{12}$ is H or $CH_3$.

This invention discloses a hydrophilic porous membrane wherein the hydrophilic porous membrane is a hydrophilic blood plasma separating membrane. Further, this invention discloses a hydrophilic porous membrane wherein surface of the hydrophilic blood plasma separating membrane is flat. Furthermore, this invention discloses a hydrophilic porous membrane wherein at least one surface of the hydrophilic blood plasma separating membrane has a plurality of minute protuberances. Further, this invention discloses a hydrophilic porous membrane wherein a grafted layer of the hydrophilic polymer is formed on the surface having the minute protuberances.

The objects of this invention described above are also accomplished by a method for the production of a hydrophilic porous membrane, which comprises preparing a hydrophobic porous membrane having as a matrix thereof a hydrophobic polymer, $\{X\}_m$, wherein m is a degree of polymerization of the hydrophobic polymer, composed of at least one kind of monomer units X as hereinabove defined and being capable of forming a hydrophobic polymer and graft polymerizing at least one kind of monomer capable of forming a hydrophilic polymer on the surface of the hydrophobic porous membrane thereby superposing thereon a graft layer of a hydrophilic polymer, $\{Y\}_n$, wherein n is a degree of polymerization of the hydrophilic polymer, composed of at least one kind of monomer units Y as hereinabove defined and being capable of forming a hydrophilic polymer and giving rise to a hydrophilic porous membrane having the monomer units Y and the monomer units X present on the membrane surface at a molar ratio, Y/X, in the range of 0.2 to 2.0 and the component polymers present in the whole web of the membrane at a weight ration, $\{Y\}_n/\{X\}_m$, in the range of 0.03 to 0.3.

This invention also discloses a method for the production of hydrophilic porous membrane whose hydrophobic polymer possesses a bubble point in the range of 0.1 to 5.0 kg/cm$^2$ and a wall thickness in the range of 50 to 500 microns and permeates water in an amount of not less than 5 ml/cm$^2$ min. under a pressure of 0.71 kg/cm$^2$.

This invention also discloses a method for the production of hydrophilic porous membrane whose component polymers are such that the subscript m has a value in the range of $10^2$ to $10^5$ and the subscript n a value in the range of 10 to $10^4$ in the respective formulas thereof. This invention further discloses a method for the production of hydrophilic porous membrane whose hydrophobic polymer is polypropylene, ethylene-propylene copolymer, or polyvinylidene fluoride. Further, this invention discloses a method for the production of hydrophilic porous membrane whose hydrophilic monomer is N,N-dimethyl acrylamide. Further, this invention discloses a hydrophilic porous membrane wherein the hydrophilic porous membrane is a hydrophilic blood plasma separating membrane.

The objects of this invention described above are also accomplished by a plasma separator which comprises accommodating a plurality of separating membrane units having at least one plasma outlet which are formed by superposing two hydrophilic blood plasma separating membranes by sealing near the circumferential portion to form a plasma flow path in a housing having a blood inlet, a blood cell outlet and plasma outlet and connecting plasma outlets of each separating membrane units with said plasma outlet of the housing, wherein the hydrophilic blood plasma separating membrane comprises a hydrophobic membrane having a matrix thereof a hydrophobic polymer, $\{X\}_m$, wherein m is a degree of polymerization of the hydrophobic polymer, composed of at least one kind of monomer units X as defined hereinabove and being capable of forming a hydrophobic polymer and a hydrophilic polymer, $\{Y\}_n$, wherein n is a degree of polymerization of the hydrophilic polymer, composed of at least one kind of monomer units Y as defined hereinabove and being capable of forming a hydrophilic polymer and graft polymerized to at least the surface of the hydrophobic porous membrane, which hydrophilic blood plasma separating membrane has the monomer units Y and the monomer units X present on the membrane surface at a molar ration, Y/X, in the range of 0.2 to 2.0 and said component polymers present in the whole web of the membrane at a weight ratio, $\{Y\}_n/\{X\}_m$, in the range of 0.03 to 0.3.

Further, this invention discloses a plasma separator, wherein said hydrophobic porous membrane possesses a bubble point in the range of 0.1 to 5.0 kg/cm$^2$ and a wall thickness in the range of 50 to 500 microns and permeates water in an amount of not less than 5 ml/cm$^2$ min. under a pressure of 0.71 kg/cm$^2$.

This invention discloses a plasma separator, wherein the component polymers are such that the subscript m has a value in the range of $10^2$ to $10^5$ and the subscript n a value in the range of 10 to $10^4$ in the respective formulas thereof. Further, this invention discloses a plasma separator, wherein the hydrophobic polymer is polypropylene, ethylene-propylene copolymer, or polyvinylidene fluoride. Further, this invention discloses a hydrophilic porous membrane whose monomer units Y are represented by the formula:

$$\begin{array}{l} -CH_2-CR^{12}- \\ \qquad\quad | \\ \qquad\quad CON(CH_3)_2 \end{array}$$

wherein $R^{12}$ is H or $CH_3$.

This invention discloses a hydrophilic porous membrane wherein the hydrophilic porous membrane is a hydrophilic blood plasma separating membrane. Furthermore this invention discloses a plasma separator,

wherein surface of the hydrophilic blood plasma membrane is flat. This invention discloses a plasma separator, wherein a sheetlike flow path forming member having a plurality of minute protuberances on the surface thereof resides between the separating membrane units comprising hydrophilic blood plasma separting membranes having a flat surface. Further, this invention discloses a plasma separator, wherein at least one surface of said hyrophilic blood plasma separating membrane has a plurality of minute protuberances. Furthermore, this invention discloses a plasma separator, wherein a graft layer of the hydrophilic polymer is formed on the surface having the minute protuberances. This invention discloses a plasma separator, wherein a sheetlike flow path forming member having a flat surface resides between the separating member units comprising hydrophilic blood plasma separating membranes having a plurality of minute protuberances on the surface thereof.

Brief Description of Drawings

Fig. 1 is a cross-sectional view which illustrates a typical blood plasma separator as one embodiment of the present invention; and

Fig. 2 is a cross-sectional view which illustrates a typical blood plasma separator as another embodiment of the present invention.

Best Mode for Carrying Out the Invention

The hydrophobic porous membrane to be used in the present invention has as a matrix thereof a hydrophobic polymer, $\{X\}_m$, composed of at least one kind of monomer units X capable of forming a hydrophobic polymer. The monomer units X of this description are those of at least one kind represented by the following formula:

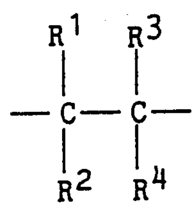

wherein, $R^1$ and $R^2$ are independently H or F, $R^3$ is H, Cl, or F, and $R^4$ is H, Cl, F, or $CH_3$, providing that $R^2$ and $R^3$ are each H unless $R^1$, $R^2$, $R^3$ and $R^4$ are each invariably F and that $R^3$ is H unless $R^3$ and $R^4$ are each equally Cl or F. Specifically, the hydrophobic polymer is either a homopolymer or a copolymer of the monomer as the unit mentioned above. Examples of the hydrophobic polymer include polyolefins partially, chlorinated or fluorinated polyolefins. Specifically, such polyolefins are polyethylene, polypropylene, polyvinylidene fluoride, polyvinylidene chloride, and polytetrafluoroethylene, for example. They may be homopolymers or copolymers of the aforementioned monomers or blends of different polymers or copolymers. The degree of polymerization of the hydrophobic polymer is in the range of $10^2$ to $10^5$ , preferably $10^3$ to $10^4$.

Desirably, the porous membrane serving as a matrix possesses a bubble point in the range of 0.2 to 5.0 kg/cm$^2$, preferably 0.3 to 2.0 kg/cm$^2$, and a wall thickness in the range of 50 to 500 microns, preferably 70 to 200 microns, and permeates water in an amount of not less than 5 ml/cm$^2$. min, preferably 10 to 40 ml/cm$^2$.min under a pressure of 0.71 kg/cm$^2$, when it is used as a plasma separator. If the bubble point of the matrix is less than 0.1 kg/cm$^2$, pore diameter increases, even after a phydrophilic membrane of the present invention is produced, so blood cells are apt to enter into the pores and to occur blockade the pores. While if it is more than 5.0 kg/cm$^2$, treatment of surface of the pore on the matrix membrane becomes difficult, what is more, after producing the phydrophilic porous membrane of the present invention, capacity for separating blood plasma of the membrane decreases. Further, if the permeation of water is less than 5 ml/cm$^2$.min, pore diameter decreases, so treatment of surface of the pore becomes difficult. The pores in the porous membrane have an average diameter in the range of 0.05 to 5.0 microns, preferably 0.2 to 1.0 microns.

Such hydrophobic porous membrane may be a membrane having flat surface or a plurality of minute protuberances on at least one surface. The hydrophobic porous membrane described above is, when the surface is flat, obtained as disclosed in U.S. Patent No. 4,734,375, for example, by mixing a hydrophobic polymer such as polyolefin with an organic filler uniformly dispersible in the hydrophobic polymer in a

molten state and readily soluble in an extracting liquid to be used and optionally further with a crystal seed forming agent, melting the resultant mixture and discharging the molten mixture through a die in the form of sheet, solidifying by cooling the sheet, and bringing the solidified membrane into contact with the extracting liquid incapable of dissolving the hydrophobic polymer thereby extracting and removing the organic filler from the membrane. Otherwise, the membrane may be obtained by preparing a solution of the hydrophobic polymer in an organic solvent and subjecting the solution to cast molding.

Where a membrane of this nature is preferable to have a plurality of minute protuberances formed on at least one of the opposite surfaces thereof can be produced as follows: In the manufacture of a permeable membrane by the solidification of an uncured dope as placed in a flat sheet, the permeable membrane is produced with the plurality of minute protuberances formed on at least one of the opposite surfaces thereby by keeping the one surface of the uncured dope in the form of the flat sheet in contact with a flat die provided with such recesses as are matched to the plurality of minute protuberances while the dope is in the process of being solidified. This method is invariably applicable wherever the porous membrane body and the portions of minute protuberances therein are formed of a material which is capable of forming a hydrophobic porous membrane and, at the same time, transforming from the fluid state to the solid state through a varying manner of solidification due to cooling, extraction with a solvent, or reversal of derivation, for example. The impartation of permeability to the material is attained indiscriminately by any of various means such as, for example, extraction with a solvent, removal of a filler, and mechanical elongation. Further, the shape of the die containing in the surface thereof a plurality of minute recesses to be used in effecting the method under discussion is not particularly restricted. The die may be in the shape of a flat sheet or in the shape of a roller. This shape may be selected so as to suit the particular process to be employed for the solidification of the uncured dope. The surface of the die containing the plurality of minute recesses gives rise to the desired minute protuberances on the surface of the porous membrane by the die surface being transferred onto the surface of the porous membrane during the solidification of the uncured dope into a membrane. The die surface, therefore, is desired to be in such a shape that the minute recesses incised therein have a depth in the range of 20 to 1,000 microns, preferably 60 to 200 microns, depending on the shape of the minute protuberances which the produced permeable membrane is desired to possess on the surface and the total area occupied by the portions of the minute recesses is in the range of 0.5 to 50%, preferably 1.0 to 20%, based on the total area of the die surface.

Now the method of this invention for the production of a porous membrane will be described more specifically below with reference to the procedure of manufacture of a flat hydrophobic porous membrane using propylene and resorting to removal of a filler. Of course, the method of this invention is not restricted in any way by this particular mode of embodiment. First, a composite material formed by mixing a polypropylene, an organic filler capable of being uniformly dispersed in the polypropylene in a molten state and readily soluble in an extracting liquid to be used, and a crystal seed forming agent is melted and discharged in the form of a flat sheet through a T die. The discharged uncured sheet of the composite material is passed through a die roll. This die roll is provided on the surface thereof with a plurality of minute recesses. When the porous membrane to be produced is desired to have minute protuberances formed only one of the opposite surfaces thereof, the discharged uncured sheet of the composite material may be passed between a die roll having the plurality of minute recesses formed on the surface thereof and a roll having a smooth surface. The uncured membrane emanating from the die roll is then brought into contact with a cool solidifying liquid to be transformed into a solidified membrane having the plurality of minute protuberances formed on the surface thereof. The solid membrane is further brought into contact with the extracting liquid incapable of dissolving the polypropylene and capable of dissolving the organic filler so as to extract the organic filler from the membrane and confer permeability upon the membrane and give virth to a flat porous membrane possessing minute protuberances.

The flat porous membrane possessing such a construction as described above may be otherwise produced as follows. On at least one of the opposite surfaces of a flat porous membrane in an already solidified state, the plurality of minute protuberances are formed by bringing this one surface of the flat porous membrane into contact with a die surface containing as many minute recesses filled in advance with a material still remaining in an uncured state and inherently possessing compatibility with or adhesiveness to the material of which the porous membrane is made and subsequently inducing solidification of the uncured material in the recesses. This method of production is invariably applicable wherever the porous membrane body is formed of a material capable of forming a porous membrane and, in the meantime, the portions of minute protuberances are made of a material which exhibits compatibility or adhesiveness to the material used for the formation of the porous membrane body and transforms from the fluid state such as melt or uncured dope to the solid state through a varying manner of solidification due to cooling or exposure to heat, light, or electron beam. This question as to what method and what conditions are to be

employed for the production of the porous membrane body is irrelevant to this applicability of the method. As the material for the protions of minute protuberances, a thermosetting resin or a resin capable of being set with light or an electron beam proves to be particularly desirable because the minute protuberances of this resin can be easily formed as attached fast to a porous membrane body by applying them to the porous membrane body and subsequently solidifying them as held in that position by application of heat or by exposure to light or an electron beam. The shape of the die possessing a surface containing a plurality of minute recesses and used in executing the method under discussion is not particularly restricted similarly to that which is used for the method of production described above. The die may be in the shape of a flat sheet or a roller. The minute recesses in the die surface are intended for the purpose of holding the uncured material destined to form protuberances and allowing the portions of the material held therein to contact the porous membrane body and, on being solidified, form desired minute protuberances on the surface of the porous membrane body as attached fast thereto. The die, therefore, is desired to possess a surface such that the minute recesses formed therein have a depth in the range of 20 to 1,000 microns, preferably 60 to 200 microns, depending on the shape of the minute protuberances desired to be formed, and the total area occupied by the portions of the minute recesses is in the range of 0.5 to 50%, preferably 1.0 to 20%, based on the total surface area of the die.

The method of production will be described more specifically below with reference to the process of manufacture of a flat porous membrane by fast attachment of minute protuberances through the medium of a thermosetting adhesive agent to a porous membrane of polypropylene to be used in an already cured state. Of course, the method of this invention is not restricted in any way by this particular mode of embodiment. First, a composite material formed by mixing a polypropylene, an organic filler capable of being uniformly dispersed in the polypropylene and being readily dissolved in an extracting liquid to be used, and a crystal seed forming agent is melted and discharged in the shape of a flat sheet through a T die. The discharged uncured sheet of the composite material is brought into contact with a cool solidifying liquid to be cooled and solidified. Then, the solidified membrane is brought into contact with the extracting liquid incapable of dissolving the polypropylene and capable of dissolving the organic filler to effect extraction of the organic filler from the membrane and consequent transformation of the solid membrane into a flat hydrophobic porous membrane destined to serve as a porous membrane body. Then, this flat porous membrane is passed between a die roll provided in the surface thereof with a plurality of minute recesses and a pinch roll. The die roll is partly immersed in a bath of thermosetting adhesive agent. By the rotation of the die roll, the thermosetting adhesive agent is allowed to adhere to the surface of the die roll. A doctor knife disposed contiguously to the surface of the die roll scrapes off the adhering thermosetting adhesive agent except for the portion thereof received inside the minute recesses. The die roll, therefore, is allowed to contact the flat porous membrane in a state in which only the minute recesses continue to hold the thermosetting adhesive agent therein. After the flat porous membrane and the die roll have been brought into mutual contact as described above, the thermosetting adhesive agent is solidified with a suitable heating means. Consequently, on the surface of the flat porous membrane, the portions of the thermosetting adhesive agent so far held inside the minute recesses of the die roll are deposited fast in a solidified state to form minute protuberances. The flat porous membrane thus produced as provided with the minute protuberances is then wound up on a takeup roll. Of course, a flat porous membrane which is provided on both the opposite surfaces thereof with a plurality of minute protuberances can be produced by causing the opposite surfaces of a flat porous membrane to contact a pair of opposed die rolls each possessing a plurality of minute recesses filled in advance with the thermosetting adhesive agent.

The hydrophilic porous membrane of the present invention is obtained by causing a hydrophilic polymer to be fixed by graft polymerization to the surface of the hydrophobic porous membrane obtained as described above and to the surface of minute pores formed in the hydrophilic porous membrane. The surface of the porous membrane and the interiors of the minute pores in the porous membrane will be collectively briefly referred to as "surface of membrane" hereinafter.

On the surface of the membrane, at least one kind of monomer units Y capable of forming a hydrophilic polymer and at least one kind of monomer units X capable of forming a hydrophobic polymer are required to be present at a molar ratio, Y/X, in the range of 0.2 to 2.0, preferably 0.4 to 1.2. If this molar ratio is less than 0.2, the surface of the matrix manifests its effect. Conversely, if this ratio exceeds 2.0, the content of the hydrophilic polymer is too high for the produced membrane to acquire sufficient compatibility to the blood.

Further in the whole web of the membrane, the weight ratio of the hydrophilic polymer, $\{Y\}_n$, to the hydrophobic polymer, $\{X\}_m$, i.e. $\{Y\}_n/\{X\}_m$, is required to fall in the range of 0.03 to 0.3 , preferably 0.05 to 0.15. If this weight ratio is less than 0.03, the impartation of hydrophilicity is not fully obtained even to the interior of the minute pores in the porous membrane. Conversely, if this weight ratio exceeds 0.3, the

amount of the hydrophilic polymer, $\{Y\}_n$, deposited on the matrix by the graft polymerization is so large that the minute pores in the membrane are swelled on the surface and are consequently diminished substantially in diameter when the membrane is swelled (in actual service), with the result that the membrane suffers the capacity thereof for blood plasma separation to be intolerably degraded.

The hyrophilic polymer, $\{Y\}_n$, to be graft polymerized to the hydrophobic porous matrix has no particular limitation except for the requirement that it should be hydrophilic. It is desired, however, not to contain an -OH group because the repression of the activation of the complement system is attained more effectively in the absence of the -OH group than in the presence thereof.

At least one kind of monomer units Y capable of forming such a preferable hydrophilic polymer as described above are those of at least one kind represented by the following formula:

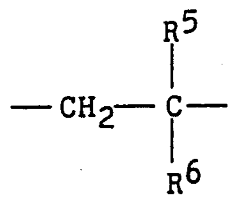

wherein $R^5$ is H or $CH_3$ and $R^6$ is

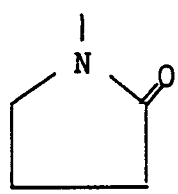,

$CONH_2$, $CONHR^7$,

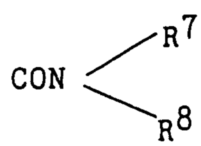

wherein $R^7$ and $R^8$ are independently an alkyl of 1 to 4 carbon atoms,

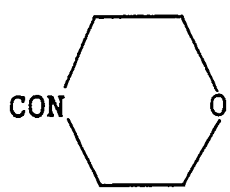

COOM, wherein M is a metallic element, $COOR^9NHR^{10}$, wherein $R^9$ is an alkylene of 1 to 4 carbon atoms and $R^{10}$ is an alkyl of 1 to 4 carbon atoms,

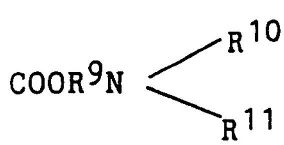,

wherein $R^9$ and $R^{10}$ have the same meanings as difined above and $R^{11}$ is an alkyl of 1 to 4 carbon atoms, or

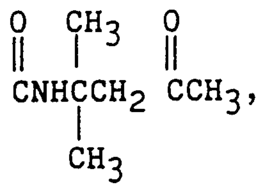

providing that $R^5$ is H where $R^6$ is

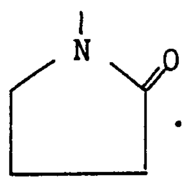

Typical examples of the hydrophilic polymer, $\{Y\}_n$, are poly-N-vinylpyrrolidone, poly(meth)acrylamides, poly-N-1ower alkyl(meth)acrylamides, poly-N,N-di-lower alkyl (meth)acrylamides, poly(meth)acryloyl morpholines, and diacetone (meth)acrylamides. Copolymers of the aforementioned monomer units are also available. The degree of polymerization of the hydrophilic polymer is in the range of 10 to $10^4$ , preferably $10^2$ to $10^4$.

Where the hydrophobic polymer, $\{X\}_m$, or the hydrophilic polymer, $\{Y\}_n$, is composed of two or more kinds of monomer units, the aforementioned molar ratio, Y/X, on the surface of the membrane will be expressed interms of the composition ratio of $\Sigma Y_i/\Sigma X_i$. Then, the weight ratio of the two component polymers, $\{Y\}_n/\{X\}_m$, in the whole web of the membrane will be expressed as (weight of hydrophilic polymer graft polymerized)/(weight of porous membrane as matrix).

The formation of a graft layer of a hydrophilic polymer by graft polymerizing a hydrophilic monomer on the surface of a hydrophobic porous membrane as a matrix can be attained by chemically graft polymerizing the hydrophilic monomer thereby forming a hydrophilic polymer moiety or by producing a radical on the chains of macromolecules of the matrix by means of electron beam, gamma ray, ultraviolet light, or low-temperature plasma and simultaneously graft polymerizing the hydrophilic monomer thereon. In this case, it is necessary that the surface structure and amount of the polymer, $\{Y\}_n$, should be controlled within the prescribed ranges by selecting the graft polymerization initiating point and the reaction conditions, for example. When a hydrophobic porous membrane having minute protuberances on the surface as a matrix, it is preferable to form graft layer of the hydrophilic polymer on the side of surface having the protuberances.

The hydrophilic porous membrane having the hydrophilic polymer graft polymerized in a prescribed amount on the surface of the hydrophobic porous membrane and the surface of the minute pores in the porous membrane has the hydrophilic polymer distributed in a low density in the web thereof in the state of high molecular mobility implying as though the polymer were partially dissolved in the blood plasma. Owing to the effect of volume of elimination and the effect of diffusion, the adsorption of blood plasma proteins and the degeneration thereof due to adsorption are repressed and the possibility of the separated blood plasma sustaining injuries is diminished.

The fact that the hydrophilic polymer is graft polymerized even inside the minute pores can be confirmed by the dyeing method or, where the porous membrane as the matrix is hydrophobic, by the observation that water drops are caused by their own weight to permeated gradually even the interior of the membrane.

When the hydrophilic porous membrane of the present invention is used as a membrane for separating blood plasma, it is preferable to permeate water in an amount of not less than 0.1 ml/cm$^2$. min. under a pressure of 0.71 kg/cm$^2$, more preferably in the range of 0.5 to 50 ml/cm$^2$. min. If the amount is less than 0.1 ml/cm$^2$. min. it is not preferable, since sufficient amount of separating blood plasma cannot be obtained. While if it exceeds 50.0 ml/cm$^2$. min., pore diameter becomes too large, thereby the blood cell component is entered into the pores on the surface of the membrane to be apt to occur blockade the pores.

When the hydrophilic porous membrane is used as a membrane for separating blood plasma, it is preferable to possess a bubble point in the range of 0.1 to 5.0 kg/cm$^2$, more preferably in the range of 0.5 to 2.0 kg/cm$^2$. If the bubble point is less than 0.1 kg/cm$^2$, pore diameter increases, so blood cell components are apt to enter into the pores on the surface of the membrane and to occur blockade the blood. Conversely, if it exceeds 5.0 kg/cm$^2$, pore diameter decrease to become lowered ability to separate

blood plasma.

The bubble point was determined by the modified method of ASTM F3l6 with a holder of stainless steel 47 mm in diameter, using isopropyl alcohol as a liquid phase. The pressure applied was gradually increased until a series of nitrogen bubbles began to rise uniformly and incessantly in the isopropyl alcohol from the central part of the filter. The pressure at which this rise of nitrogen bubbles started was reported as the bubble point.

The molar ratio, Y/X, of the component polymers on the surface of the membrane was determined by the calculation of the surface elemental composition with ESCA (produced by Japan Electron Optics Laboratory Co., Ltd. and marketed under product code of "JPS-90SX") under a condition of 90° of incidence angle. The weight ratio, $\{Y\}_n/\{X\}_m$, was determined by the weight method.

The hydrophilic porous membrane which is obtained as described above finds utility in a varying application which the attributes of the porous membrane body fit. Particularly it manifests an outstanding performance when it is incorporated as a blood plasma separating membrane in a module as illustrated below.

Fig. 1 illustrates a typical blood plasma separator as one embodiment of this invention. A case comprises a cylindrical case body 24 provided in the central part of an upper plate thereof with a blood inlet 21, near the circumferential part of the upper plate thereof with blood plasma outlets 22, and on the lateral wail thereof with a blood outlet 23 and a bottom lid 25 lasteral wall thereof with a blood outlet 23 and a bottom lid 26 fitted on the periphery thereof with an O-ring 25. Inside this case, a plurality of membrane units 31 which are each formed by interposing a circular plasma flow path forming member 27 made of screen mesh or non-woven fabric and provided at the center thereof with an opening part 28 and near the outer circumference thereof with plasma passing holes 29 between two horizontally parallelled hydrophilic porous membranes l0a, 20b provided on the outsides thereof with a plurality of minute protuberances 20 are superposed one over another as severally interposed between flat sheetlike flow path regulating members 32 which are each provided with a central opening part 28 and plasma passing holes 29 correspondingly to those of the aforementioned units 31 and have seal members 30 circumferentially attached one each to the plasma passing holes 29.

The seal members 30 integrally join the membrane units 31 and the flat sheetlike flow path regulating members 32. The minute protuberances 20 existing on the outersides of the flat porous membranes 10a, 10b and the flat sheetlike flow path regulating members 32 disposed as opposed to the minute protuberances 20 cooperate to support the porous membranes l0a, l0b and prevent them from being deformed. The spaces intervening among the minute protuberances 20 permit passage of the body fluid. The separation of the blood plasma from the blood by the use of this blood plasma separator is accomplished by introducing the blood from a human body through the body fluid inlet 21 into the central opening part 28, and advanced through the body fluid flow path formed by the spaces intervening between the minute protuberances 20. The blood in the process of flowing through the flow path is allowed to permeate the blood plasma separating membranes 10a, 10b. The blood plasma which has been separated as a filtrate departs from the interior of the membrane units 31, flows through the filtrate passing holes 29, and flows out of the blood plasma outlet 22. In the meantime, the blood separated by the filtration is discharged through the body fluid outlet 23.

When the flat separating membrane of this invention provided with protuberances as described above is utilized in the blood plasma separator as illustrated in Fig. 1, it is preferable to fulfil more restrictive conditions as follows. To be specific, in the flat separating membrane provided with protuberances, the minute protuberances 20 are preferable to be such that the height thereof is in the range of 20 to 200 microns, the diameter at the base thereof in the range of 100 to 1,000 microns, the interval between the apexes thereof in the range of 30 to 2,000 microns, and the total area occupied by the protuberances in the range of 3 to 20%, based on the total surface of the membrane. Preferably, the height is in the range of 50 to 100 microns, the diameter at the base in the range of 200 to 500 microns, the interval between the apexes in the range of 500 to 1,000 microns, and the total area of the minue protuberances in the range of 5 to 15%, based on the total area of the membrane.

Specifically, the height of the individual minute protuberances on the surface of the porous membrane constitutes itself an important factor for defining the thickness of the flow path for the body fluid. From the standpoint of the filtration engineering, if the height of the minute protuberances is less than 20 microns, the thickness of the flow path for the body fluid is so small as to induce heavy pressure loss. If this height exceeds 200 microns, the shearing speed cannot be increased appreciably and sufficient filtration of the body fluid cannot be obtained.

All the factors considered, it is found that the height of the minute protuberances is desired to fall in the range of 20 to 200 microns. Though this height is desired to be fixed, the constancy of this height is not an

indispensable requirement. Optionally, it may be varied stepwise along the direction of the flow of the body fluid.

It has been also found that the interval between the individual minute protuberances on the surface of the filtering membrane constitutes itself one of the most important factors for the formation of a uniform and stable flow path for the body fluid. If the interval between the minute protuberances 2 is less than 300 microns, the available surface area of the membrane is descreased and the pressure loss is increased excessively. Conversely, if this interval exceeds 2,000 microns, the membrane sustains minute distortions, fails to retain a uniform and stable flow path for the body fluid, and manifests no sufficient performance. The interval between the minute protuberances 20, therefore, is desired to be in the range of 300 to 2,000 microns, preferably 500 to 1,000 microns. The minute protuberances are desired to be distributed uniformly throughout the entire surface of the filtering membrane. A slight deviation from this uniformity is permissible, however, where this deviation avoids impairing the uniform flow of the body fluid.

If the diameter of the minute protuberances at the base is less than 100 microns, the minute protubrances form a uniform flow path for the blood with difficulty. Conversely if the diameter exceeds 1,000 microns the minute protuberances occupy an unduly large surface area. Thus, the diameter is desired to be in the range of 100 to 1,000 microns, preferably 200 to 500 microns.

If the total area occupied by the minute protuberances is less than 3%, the minute protuberances have the possibility of ceasing to retain a uniform flow path for the body fluid perfectly under harsh conditions of filtration. If the total area exceeds 20%, the device requires an unduly large volume to offer an amply large available area of filtration. Thus, the total area is desired to be in the range of 3 to 20%, preferably 5 to 15%.

The flat sheetlike flow path regultaing members are preferable to be made of a rigid material so as to ensure formation of highly uniform and stable flow path for the body fluid with a small wall thickness. The term "rigid material" as used herein referes to what avoids sustaining depression or other similar deformation on contact with the protubrances on the memberane surface. Specifically, the rigidity of the material must exceed a Brinell number of 10. Examples of the material possessing such a Brinell number include polyethylene, polypropylene, polyester, and polycarbonate.

Fig. 2 illustrates a typical blood plasma separator as one embodiment of this invention as disclosed in U.S.P. 4,597,868. A case comprises a cylindrical case body 54 provided in the central part of an upper plate thereof with a blood inlet 51, near the circumferential part of the upper plate thereof with blood plasma outlets 52, and on the lateral wall thereof with a blood outlet 53 and a bottom lid 56 fitted on the periphery thereof with an O-ring 55. Inside this case, a plurality of membrane units 51 which are each formed by interposing a circular plasma flow path forming member 57 made of screen mesh or non-woven fabric and provided at the center thereof with an opening part 58 and near the outer circumference thereof with filtrered body fluid passing holes 59 between two horizontally parallelled hydrophilic porous membranes 40a, 40b are superposed one over another as severally interposed between flow path regulating members 62 having a plurality of protuberances which are each provided with a central opening part 58 and plasma passing holes 59 correspondingly to those of the aforementioned units 51 and have seal members 60 circumferentially attached one each to the plasma passing holes 59.

Now, the present invention will be described more specifically below with reference to working examples.

The ability of collecting blood plasma was determined of a mini-module of a given membrane having an available surface area of 24 $cm^2$ (6 cm x 4 cm) using bovine blood adjusted to a hematocrit value of 40 and a total protein concentration of 5.8 g/dl under the conditions of a shear speed of 300 ($sec^{-1}$) and a permeation pressure (TPM) of 2000 to 2666 Pa (15 to 20 mmHg).

Examples 1 to 3

With a twin-screw extruder (produced by Ikegai Iron works, Ltd. and marketed under product code of "PCM-30"), 100 parts by weight of polypropylene (polymerization degree about 1,200), 400 parts by weight of liquid paraffin, and 0.3 part by weight of 1,3,2,4- bis(para-ethylbenzylidene)sorbitol as a crystal seed forming agent were melted and mixed and then extruded. The extruded mixture was then pelletized. In the same extruder, the pellets were melted at l80 °C. The molten mixture was extruded through a T die 0.6 mm in slit width at a rate of 200 g/min. into the ambient air. The extruded sheet was brought into contact with a cooling roll disposed below the T die and kept at a surface temperature of 35 °C to be solidified by cooling. The solidified sheet was taken up on a winding roll. A rectangular piece measuring 130 mm x 250 mm was cut from the sheet, fixed in the longitudinal and lateral directions, immersed twice each for 10 minutes in 1,1,2- trichloro-l,2,2-trifluoroethane at a temperature of 25 °C to effect extraction of the liquid paraffin, and

then subjected to a heat treatment in the air at l35°C for two minutes. The porous polypropylene membrane consequently obtained was found to possess a bubble point of 1.1 kg/cm$^2$ , an average pore diameter of 0.5 micron, and a wall thickness of 130 microns.

This porous membrane was set in a reaction vessel having an inner diameter of 120 mm and, in an atmosphere of argon gas, exposed for 10 seconds to a low-temperature plasma generated by the application of an electric power of 50 W with electrodes connected to a high frequency (13.56 MHz) generator under 6.7 Pa (0.05 Torr). After the exposure to the plasma, N,N-dimethyl acrylamide gas was introduced into the reaction vessel and graft polymerized for a varying period under 106.7 Pa (0.8 Torr).

The hydrophilic porous membranes consequently obtained were tested for the molar ratio, Y/X, on the membrane surface, the weight ratio, $\{Y\}_n/\{X\}_m$, in the whole membrane, and the ability to collect blood plasma. The results are shown in Table 1.

Controls 1 to 3

Hydrophilic porous membranes differing in the molar ratio, Y/X, and the weight ratio, $\{Y\}_n/\{X\}_m$, were synthesized by following the procedure of Example 1, except that the reaction conditions were changed and were tested for ability to collect blood plasma. The results are shown in Table I. The hydrophilic blood plasma separating membranes obtained herein were inferior in performance to those of Examples 1 to 3.

Examples 4 to 6 and Controls 4 to 6

Hydrophilic porous membranes possessing different molar ratios, Y/X, on membrane surface were produced by following the procedure of Example 1, except that a polypropylene sheet (wall thickness 50 microns) was used as a matrix instead. Platelet adhesion onto polymer surfaces was studied. This test was carried out by keeping a sample in contact with a platelet rich plasma (1 x $10^8$ platelet/ml) for 30 minutes, rising with physiological saline solution, and fixing, and observing the sample under a scanning electron microscope to count the number of adhering blood platelets in five fields (1,000 magnification) of view. Separately, a polypropylene sheet used as a matrix and a polypropylene sheet treated with low temperature argon plasma were subjected to the same test. The results are shown in Table 2. The morphological changes of adhering blood platelets were classified under three types, Type I, Type II, and type III, as defined below, depending on the condition of progress of change.

Type I: Blood platelets undergone change from normal discs to spheres with growth of three or four pseudopodia and believed to adhere rather weakly to the surface of sample.

Type II: Blood platelets undergone change to a point where they grew more than several pseudopodia up to half of which involved expansion of spores, and believed to adhere strongly to the surface of sample.

Type III: Blood platelets undergone change to a point where those having expanded thin spores to more than half the length of pseudopodia were allowed to expand the spores substantially completedly and assume the appearance of a near circle and believed to adhere perfectly to the surface of sample.

Examples 7 and 8 and Controls 7 and 8

Two hydrophilic blood plasma separating membranes, one possessing a molar ratio, Y/X, of 0.3 and a weight ratio, $\{Y\}_n/\{X\}_m$, of 0.07 and the other possessing a molar ratio, Y/X, of 0.5, and a weight ratio, $\{Y\}_n$ $\{X\}_m$, of 0.12 were synthesized by following the procedure of Example I. They were subjected to an in vitro blood plasma separation test to determine the ratio of recovery of fibrinogen and the ratio of recovery of coagulating activity of factor VIII (F. VIII:C) as indexes for the ratio of recovery of coagulation factor in the permeating blood plasma and the $C_3$a concentration in the permeating blood plasma as an index for the activation of the complement system. The $C_3$a was determined using RIA kit of Amersha, Inc. COATEST factor VIII:C was determined by chromophorib synthetic substrate method (Kabi Vitrum AB, Sweden). It is index showing how degree of the activity the blood coagulating system in the separated plasma has, and it is suitable for the plasma separating membrane as large as possible.

The test was carried out using a disclike module of an available surface area of 116 cm$^2$ under the conditions of a permeation pressure (TMP) of 3332 Pa (25 mmHg) and a temperature of 37°C.

For comparison, a porous membrane of ungrafted polypropylene (pore diameter 0.45 micron and wall thickness 140 microns) and a membrane of cellulose acetate (pore diameter 0.45 micron and wall thickness 150 microns) were similarly tested. The data on membrane surface structure obtianed during the initial

stage of permeation (10 minutes following the start of separation), a period in which the adsorption and activation of blood plasma proteins, etc. are liable to be affected by the surface condition, are shown in Table 3.

The results of the test indicate that the hydrophilic blood plasma separating membranes produced in the working examples showed higher ratios of recovery of fibrinogen and F.VIII:C, caused less activation of the complement system, and exhibited better compatibility blood than the membranes prepared for comparison.

Table 1

| | Y/X | $(Y)_n/(X)_m$ | Hydrophilicity | Bubble point (kg/cm²) | Ability to collect blood plasma, Q (ml/min.cm². Pa) |
|---|---|---|---|---|---|
| Example 1 | 0.41 | 0.085 | + | 1.12 | $1.65\ 10^{-3}$ |
| Example 2 | 0.45 | 0.10 | + | 1.15 | $1.8\ 10^{-3}$ |
| Example 3 | 0.6 | 0.14 | + | 1.18 | $1.88\ 10^{-3}$ |
| Control 1 | < 0.2 | 0.02 | - | 1.10 | |
| Control 2 | 1.1 | 0.52 | + | 0.86 | $6\ 10^{-4}$ |
| Control 3 | 3.6 | 0.92 | + | more than 5.0* | $3.75\ 10^{-4}$ |

Y; 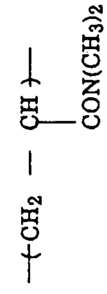

X; 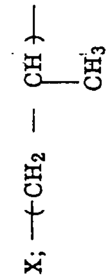

* Bubble point exceeding 5.0 could not be measured.

Table 2

| | Y/X | Number of adhering blood platelets Type I | Type II | Type III |
|---|---|---|---|---|
| Example 4 | 0.28 | 84 | 5 | 9 |
| Example 5 | 0.42 | 25 | 1 | 1 |
| Example 6 | 0.77 | 38 | 0 | 0 |
| Control 4 | (Polypropylene) | 301 | 48 | 111 |
| Control 5 | (Polypropylene treated with plasma) | 345 | 42 | 68 |
| Control 6 | 0.16 | 162 | 11 | 22 |

Table 3

| | Example 7 | Example 8 | Control 7 (Polypropylene membrane) | Control 8 (Cellulose acetate membrane) |
|---|---|---|---|---|
| Pore diameter (micron) | 0.45 | 0.45 | 0.45 | 0.45 |
| Wall thickness (micron) | 140 | 140 | 140 | 150 |
| X/Y | 0.3 | 0.5 | | |
| $-(Y)_n-/-(X)_m-$ | 0.07 | 0.12 | | |
| Bubble point ($kg/cm^2$) | 1.12 | 1.15 | 1.10 | 0.97 |
| $\frac{C_3a \text{ in permeating blood plasma}}{C_3a \text{ in blood plasma before separation}}$ | 1.9 | 3.4 | 5.6 | 21.2 |
| Ratio of recovery of fibrinogen (%) | 93 | 91 | 54 | 91 |
| Ratio of recovery of F.VIII:C (%) | 90 | 91 | 73 | 99 |
| Ratio of recovery of total proteins (%) | 92 | 92 | 91 | 89 |

Examples 9 and 10 and Controls 9 and 10

Porous membranes of polypropylene prepared by following the procedure of Example 1 were irradiated with the electron beam from an electron accelerator (applied voltage 1.5 MeV and electron gun current 1 mA) and immersed in a solution consisting of 10 parts by weight of N,N-dimethyl aminoethyl acrylate and 90 parts by weight of acetone to undergo graft polymerization at 50°C for 30 minutes. The membranes were washed with methanol for 40 hours, dried, and subjected to various tests. The results are shown in Table 4.

Examples 11 and 12 Controls 11 and 12

A solution of 18 parts by weight of polyvinylidene fluoride powder (produced by Mitsubishi Petro-Chemical Co., Ltd. and marketed under trademark designation of "Kynar K30l") (polymerization degree about 5,000) in 73.8 parts by weight of acetone and 8.2 parts by weight of dimethyl formamide was cast on a polyethylene terephthalate film. The resultant composite film was immersed in a l,l,2-trichloro-l,2,2-trifluoroethane bath for five minutes and then dried, to afford a porous membrane of polyvinylidene fluoride possessing a wall thickness of 135 microns, an average pore diameter of 0.5 micron and 0.98 $kg/cm^2$ of bubble point. This membrane was irradiated with an electron beam in the same manner as in Example 9, placed in a closed container which was evacuated of oxygen and then filled with N,N-dimethyl acrylamide in gaseous form, and left therein undergoing graft polymerization under the conditions of 80 to 106.7 Pa (0.6 to 0.8 Torr) and 50°C. The membrane was washed, dried, and subjected to various tests. The results are shown in Table 4.

Examples 13 and 14 and Controls 13 and 14

A porous membrane possessing an average pore diameter of 0.6 micron, a wall thickness of 130 microns, and 0.95 $kg/cm^2$ of bubble point was obtained by following the procedure of Example 1, except that 60 parts by weight of polypropylene (produced by Mitsui Toatsu Chemicals Inc. and marketed under product code of "J3H") (polymerization degree about 1,200) and 40 parts by weight of random polymer of ethylene-propylene (produced by Mitsui Toatsu Chemicals Inc. and marketed under product code of "GEBG") (polymerization degree about 4,000) were used instead. The porous membrane was impregnated with a solution containig 15 parts by weight of N-vinyl pyrrolidone, 80 parts by weight of acetone, and 5 parts by weight of benzophenone as a photosensitizer. The impregnated porous membrane was nipped between two opposed glass sheets to prevent vaporization of the solvent and graft polymerized for 0.4 hour under a current of nitrogen gas by irradiation with UV from a UV irradiation device (produced by Nippon Bunka Seiko KK.). The membrane was washed for two days with a Soxhlet extractor using methanol as a solvent and then evaluated. The results are shown in Table 4.

Examples 15 and 16 and Controls 15 and 16

A porous membrane possessing an average pore diameter of 0.6 micron and a wall thickness of 130 microns was obtained by following the procedure of Example 1, except that 71 parts by weight of polypropylene (produced by Mitsui Toatsu Chemicals Inc. and marketed under produce code of "J3H") (polymerization degree about 1,200) and 29 parts by weight of random polymer of ethylenepropylene (produced by Mitsui Toatsu Chemicals Inc. and marketed under product code of "GEBG") (polymerization degree about 4,000) were used instead. The porous membrane was set in a cylindrical plasma generating chamber 300 mm in inside diameter and 300 mm in length and subjected to 30 seconds' treatment in vacuo ultraviolet light in an atmosphere of 6.7 Pa (0.05 Torr) in such a manner that the short-wavelength ultraviolet light (vacuo ultraviolet light) generated by the low temperature plasma would reach the membrane surface and avoid directly contacting the anodic plasma ray. Then, the argon gas pressure was lowered to 1.3 Pa (0.01 Torr) and N,N-dimethyl acrylamide was brought into contact with the membrane under 80 Pa (0.6 Torr) to effect graft polymerization. The membrane was washed, dried, and then subjected to various tests. The results are shown in Table 4

Example 17

A solution of 20 parts by weight of polyvinylidene fluoride powder (produced by Mitsubishi Petro-Chemicals Inc. and marketed under trademark designation of "Kynar K301") (polymerization degree about 5,000) in 80 parts by weight of acetone was cast on a polypropylene film. The resultant composite film was immersed in a 1,1,2-trichloro-1,2,2trifluoroethane bath for five minutes and then dried. Consequently, a porous membrane of polyvinylidene fluoride possessing a wall thickness of 125 microns and an average pore diameter of 0.5 micron was obtained. This porous membrane was irradiated with 2 Mrads of gamma ray and was graft polymerized by contact with N,N-dimethyl acrylamide gas under the conditions of 80 Pa (0.6 Torr) and 25°C in the absence of oxygen. The graft polymerized porous membrane was treated with buytyl acrylate gas (266.6 Pa : 2.0 Torrs) for further growth of the graft chains. The membrane was washed and dried and subjected to various tests. The results are shown in Table 4.

Control 17

Graft polymerization was carried out by following the procedure of Example 17, except that butyl acrylate alone was used as a monomer. The membrane consequently obtained was subjected to the same tests. The results are shown in Table 4.

Example 18

A porous membrane possessing an average pore diameter of 0.6 micron and a wall thickness of 130 microns was obtained by following the procedure of Example 1, except that 60 parts by weight of polypropylene (produced by Mitsui Toatsu Chemicals Inc. and marketed under product code of "J3H") (polymerization degree about 1,200) and 40 parts by weight of random polymer of ethylene-propylene (produced by Mitsui Toatsu Chemicals Inc. and marketed under product code of "GEBG") (polymerization degree about 4,000) were used instead. The porous membrane was immersed in a methanol 10% acryloyl morpholine solution for five minutes and then dried and irradiated with the electron beam from an electron accelerator (applied voltage 1.5 MeV and electron gun current 1 mA). The membrane was washed and dried and subjected to various tests. The results are shown in Table 4.

Table 4

| | Reaction time | Y/X | $-(Y)_n-/-(X)_m-$ | Bubble Point ($kg/cm^2$) | Ratio of recovery of F. VIII:C | Ability to collect blood plasma, Q [$ml/min.cm^2.Pa$] | Hydrophilicity |
|---|---|---|---|---|---|---|---|
| Example 9 | 6.0 min. | 0.6 | 0.09 | 1.18 | 93 | $1.58\ 10^{-3}$ | + |
| " 10 | 10.0 min. | 1.1 | 0.14 | 1.23 | 94 | $1.43\ 10^{-3}$ | + |
| Control 9 | 1.0 min. | 0.3 | 0.02 | 1.12 | 79 | $1.65\ 10^{-3}$ | - |
| " 10 | 60.0 min. | 3.2 | 0.43 | more than 5.0* | 24 | $3.75\ 10^{-4}$ | + |
| Example 11 | 10.0 min. | 1.2 | 0.12 | 1.17 | 93 | $1.88\ 10^{-3}$ | + |
| " 12 | 15.0 min. | 1.4 | 0.16 | 1.20 | 93 | $1.65\ 10^{-3}$ | + |
| Control 11 | 1.0 min. | 0.4 | 0.02 | 0.97 | 91 | $1.73\ 10^{-3}$ | - |
| " 12 | 60.0 min. | 4.0 | 0.51 | more than 5.0* | 34 | $6\ 10^{-4}$ | + |
| Example 13 | 10.0 min. | 1.0 | 0.09 | 1.10 | 89 | $1.58\ 10^{-3}$ | + |
| " 14 | 20.0 min. | 1.5 | 0.15 | 1.31 | 88 | $1.58\ 10^{-3}$ | + |
| Control 13 | 2.0 min. | 0.3 | 0.02 | 1.02 | 73 | $1.58\ 10^{-3}$ | - |
| " 14 | 60.0 min. | 8.0 | 0.32 | 4.6 | 32 | $5.25\ 10^{-4}$ | + |
| Example 15 | 5.0 min. | 0.8 | 0.12 | 0.98 | 92 | $1.73\ 10^{-3}$ | + |
| " 16 | 7.0 min. | 1.3 | 0.15 | 1.02 | 92 | $1.73\ 10^{-3}$ | + |
| Control 15 | 0.5 min. | 0.3 | 0.02 | 0.96 | 78 | $1.65\ 10^{-3}$ | - |
| " 16 | 20.0 min. | 3.6 | 0.28 | 2.12 | 46 | $6\ 10^{-4}$ | + |
| Example 17 | 10.0 min. | 0.3 | 0.08 | 1.06 | 93 | $1.88\ 10^{-3}$ | + |
| Control 17 | 10.0 min. | 1.5 | 0.10 | 1.10 | 86 | $1.73\ 10^{-3}$ | - |
| Example 18 | 0.2 min. | 0.7 | 0.06 | 1.18 | 90 | $1.8\ 10^{-3}$ | + |

* Bubble point exceeding 5.0 $kg/cm^2$ could not be measured.

Example 19

With a twin-screw extruder (produced by Ikegai Iron works, Ltd. and marketed under product code of "pCM-30"), 100 parts by weight of polypropylene (mixture of polypropylene having polymerization degree of 1,200 with polypropylene having polymerization degree of 4,000, weight ratio 100 to 40), 400 parts by weight of liquid paraffin (a number average molecular weight of 324), and 0.3 part by weight of 1,3,2,4-dibenzylidene sorbitol as a crystal seed forming agent were melted and mixed and then pelletized In the same extruder, the pellets were melted at 150° to 200°C. The molten mixture was extruded through a T die into the ambient air, and then dropped to a guide roller, disposed below the T die and introduced into a

cooling solidifying solution comprising polyethylene glycol to be solidified. The solidified sheet was immersed in 1,1,2,trichloro-1-2-2-trifluoroethane at a temperature of 25°C to effect extraction of the liquid paraffin, and then subjected to a heat treatment in the air at l35°C for two minutes to obtain a polyrpopylene membrane having a surface smoothness. The porous membrane consequently obtained was stick with refainer plate (silicone rubber measuring 200 mm x 200 mm, having hemispherical inpression of 0.3 mm in diameter and 0.15 mm in depth provided in 2% of total area occupied by the protuberances, based on the total surface of the plate), which was applied to a polyurethane type adhesive agent (produced by Nippon plyurethane Co., Ltd.), then the adhesive agent remaining on the surface thereof was shaved with a doctor knife and then the adhesive agent was introduced into the hemisherical impressions thereof. After 20 minutes, the porous membrane was peeled off from the plate. The membrane consequently obtained was a hydrophobic porous membrane having protubers which formed hemispherical protruberance which formed hemispherical protruberances having 0.28 mm in diametr and 0.12 mm in depth uniformly on the surface thereof. This porous membrane had 0.45 microns in pore diameter and bubble point of 1.14 kg/cm$^2$. The hydrophilic porous membrane was obtained by graft polymerization of N,N-dimethylacrylamide in the same manner as in Example l.

The hydrophilic porous membrane consequently obtained was tested for the molar ratio, Y/X, on the membrane surface, the weight ratio, $\{Y\}_n/\{X\}_m$, in the whole membrane. The results are shown in Table l. Further, this membrane was tested for complement activating component $C_3a$. The results are also shown in Table 5.

Example 20

On the surface of a polypropylene porous membrane obtained by a similar method to Example 19, an ultraviolet curing resin (produced by Dai Nippon Inc. Co., Ltd.) was printed by the use of a rotary screen printer (produced by Nippon Bunka Seiko Co., Ltd.) comprising print plate having pores o.f 0.5 mm in diameter and 0.18 mm in depth provided in 2% of total area occupied by the protuberances, based on the total surface of the plate). Immediately after print, ultra violet was exposed thereto for 10 minutes using 2 Kw of ultraviolet lamp (Nippon Bunka Seiko Co., Ltd.). The membrane consequently obtained was a hydrophobic porous membrane having protuberances which formed hemisherical protuberances having 0.52 mm in diameter and 0.12 mm in height uniformly on the surface thereof. This porous membrane had 0.45 microns in pore diameter and bubble point of 1.14 kg/cm$^2$.

The hydrophilic porous membrane was obtained by graft polymerization of N,N-dimethylacrylamide in the same manner as in Example l.

The hydrophilic porous membrane consequently obtained was tested for the molar ratio, Y/X, on the membrane surface, the weighr ratio, $\{Y\}_n/\{X\}_m$, in the whole membrane. The results are shown in Table 1. Further this membrane was tested for complement activitating component, $C_3a$. The results also shown in Table 5.

Table 5

| | Amount of priming (ml) | Condition of filtration: Amount of blood flow (ml/min.) | Condition of filtration: Pressure of diaphgragna (mmHg) | Y/X | $(Y)_n/(X)_m$ | Bubble point ($kg/cm^2$) | $C_{3a}$ in permeating blood plasma / $C_3$ in blood plasma before separation |
|---|---|---|---|---|---|---|---|
| Example 19 | 40 | 100 | 40 | 0.39 | 0.06 | 1.16 | 2.5 |
| Example 20 | 40 | 100 | 40 | 0.57 | 0.11 | 1.20 | 2.0 |

Example 21 and 22

Permeabable membrane units were formed by combinating two sheets of hydrophilic blood plasma separating membranes having protuberances obtained from Examples 19 and 20 and then sealing between

the outer and the interal circumference thereof by a heat seal method (at l40 °C for 2 secnds) or an ultrasonic fusion method (using an ultrasonic welder model 8700 produced by Blanson). A flat board type flow part regulating member has 102 mm in outer diameter and 22 mm in pore diameter of central opening portion. The material and Brinell hardness are as shwon in Table 6.

The permeable membrane units and the flat blood type flow path regulating members are laminated in turn with a hot melt adhesive agent (produced by Toa Gosei kagaku Kogyo Co., Ltd. marketed under product code PPET-l009) as shown in Fig. 1, it was put away in a cylindrical container, and then a planger made of polycarbonate was inserted therein via an O-ring made of silicone rubber to obtain a blood plasma separator.

In the device as described above, anticoagulant (ACD solution) added human blood having a hematocrit value of 38%, at temperature of 37 °C was flowed at flow rate of 50ml per minute to measure an amount of filtration of blood plasma, $Q_F$. As the numeral which indicates a size of device, a thickness of the device laminated portion (thickness of the device except for the cylindrical container and the plunger) was measured. An amount of priming, Pv, in blood side was also measured. These results are shown in Table 6.

Table 6

| | Example 21 | Example 22 |
|---|---|---|
| Filtration membrane | | |
| Kind | having protuberance | having protuberance |
| shape of protuberance | | |
| height (micron) | 70 | 70 |
| diameter (micron) | 300 | 300 |
| ocupied area (%) | 2 | 15 |
| Flat board type | | |
| flow regulting member | polycarbonate | polycarbonate |
| material | 40 | 40 |
| Brinell hardness | 50 | 50 |
| Thickness (micron) | 11 | 11 |
| Number of laminating (layer) | 0.12 | 0.12 |
| Effective area ($m^2$) | 10 | 10 |
| Thickness (mm) | 20 | 19 |
| $P_v$ (ml) | 22 | 20 |
| $Q_F$ (ml/min.) | | |

Industrial Applicability

As described above, this invention is directed to a hydrophilic porous membrane comprising a hydrophobic porous membrane having as a matrix thereof a hydrophobic polymer, $\{X\}_m$, wherein m is a degree of polymerization of the hydrophobic polymer, composed of at least one kind of monomer units X capable of forming a hydrophobic polymer and a hydrophilic polymer, $\{Y\}_n$, wherein n stands for the degree of polymerization of the hydrophilic polymer, composed of at least one kind of monomer units Y capable of forming a hydrophilic polymer and graft polymerized to at least the surface of the hydrophobic porous membrane, which hydrophilic porous membrane has the monomer units X and the monomer units Y present on the membrane surface at a molar ratio, Y/X, in the range of 0.2 to 2.0 and the component

polymers present in the whole web of the membrane at a weight ratio, $\{Y\}_n/\{X\}_m$, in the range of 0.03 to 0.3 and to method for the production of the membrane. Since the hydrophilic polymer is present in the membrane in a low density in the state of high molecular mobility implying as though the polymer were partially dissolved in the blood plasma, when it is used as a hydrophilic blood plasma separating membranes, it manifests an effect of volume of elimination and an effect of diffusion and consequently represses the adsorption of blood plasma proteins and the degeneration of the proteins by adsorption and diminishes the possibility of the separated blood plasma sustaining injuries. As the result, the membrane recovers the coagulation factor in the blood plasma in a high ratio, avoids appreciably activating the complement system, and exhibits high compatibility to blood. As a porous membrane, therefore, the membrane of this invention manifests an outstanding effect in curing diseases due to abnormal immunity and in collection of blood plasma for transfusion. Owing to the excellent compatibility to blood, the hydrophilic porous membrane of this invention is also useful as a blood treating membrane in artificial kidneys and artificial livers and as a porous medium for adsorption.

## Claims

**1.** A hydrophilic porous membrane comprising :

a) a porous membrane having as a matrix thereof a hydrophobic polymer, $\{X\}_m$, wherein $_m$ is the degree of polymerization of said hydrophobic polymer, composed of at least one kind of monomer units X of formula :

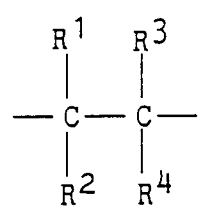

wherein, $R^1$ and $R^2$ are independently H or F, $R^3$ is H, Cl, or F, and $R^4$ is H, Cl, F, or $CH_3$, providing that $R^2$ and $R^3$ are each H unless $R^1$, $R^2$, $R^3$, and $R^4$ are each invariably F and that $R^3$ is H unless $R^3$ and $R^4$ are each equally Cl or F, said monomer units being capable of forming a hydrophobic polymer; and

b) a hydrophilic polymer, $\{Y\}_n$, wherein $_n$ is the degree of polymerization of said hydrophilic polymer, composed of at least one kind of monomer units Y of formula :

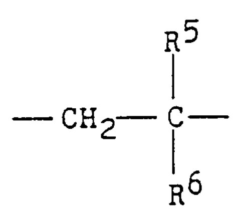

wherein $R^5$ is H or $CH_3$ and $R^6$ is

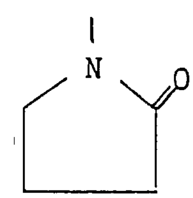 ,

$CONH_2$, $CONHR^7$,

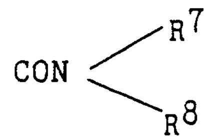

wherein $R^7$ and $R^8$ are independently an alkyl of 1 to 4 carbon atoms,

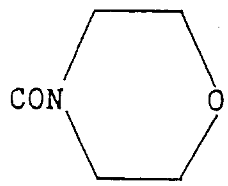 ,

COOM, wherein M is a metallic element, $COOR^9NHR^{10}$, wherein $R^9$ is an alkylene of 1 to 4 carbon atoms and $R^{10}$ is an alkyl of 1 to 4 carbon atoms,

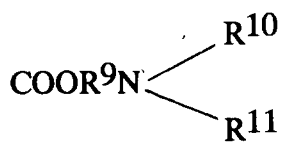 ,

wherein $R^9$ and $R^{10}$ have the same meanings as defined above and $R^{11}$ is an alkyl of 1 to 4 carbon atoms, or

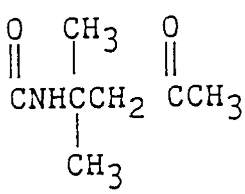 ,

providing that $R^5$ is H when $R^6$ is

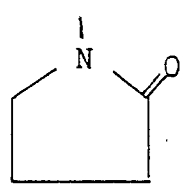 ,

said monomer units Y being capable of forming a hydrophilic monomer, and graft polymerized to at least the surface of said porous membrane ;

and having said momer units Y and said monomer units X present on the membrane surface at a molar ratio, Y/X, in the range of 0.2 to 2.0 and said component polymers present in the whole web of the membrane at a weight ratio, $(Y)_n/(X)_m$ in the range of 0.03 to 0.3.

2. A hydrophilic porous membrane according to Claim 1, wherein said component polymers are such that the subscript m has a value in the range of $10^2$ to $10^5$ and the subscript n a value in the range of 10 to $10^4$ in the respective formulas thereof.

3. A hydrophilic porous membrane according to Claim 1 or 2, wherein said monomer units Y are represented by the formula:

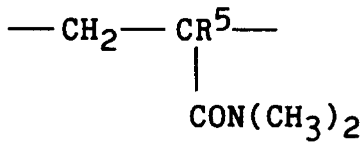

wherein $R^5$ is H or $CH_3$.

4. A hydrophilic porous membrane according to any one of Claims 1 to 3, wherein said hydrophilic porous membrane possesses a bubble point in the range of 0.1 to 5.0 kg/cm$^2$ and a wall thickness in the range of 50 to 500 microns and permeates water in an amount of not less than 5 ml/cm$^2$. min. under a pressure of 0.71 kg/cm$^2$.

5. A hydrophilic porous membrane according to any one of Claims 1 to 4, wherein said hydrophobic polymer is polypropylene, ethylene-propylen copolymer or polyvinylidene fluoride.

6. A hydrophilic porous membrane according to any one of Claims 1 to 5, wherein said hydrophilic porous membrane is a hydrophilic blood plasma separating membrane.

7. A hydrophilic porous membrane according to Claim 6, wherein surface of said hydrophilic blood plasma membrane is flat.

8. A hydrophilic porous membrane according to Claim 6, wherein at least one surface of said hydrophilic blood plasma separating membrane has a plurality of minute protuberances.

9. A hydrophilic porous membrane according to Claim 8, wherein a graft layer of said hydrophilic polymer is formed on the surface having the minute protuberances.

10. A plasma separator which comprises accommodating a plurality of separating membrane units having at least one plasma outlet which are formed by superposing two hydrophilic blood plasma separating membranes and sealing near the circumferential portion to form a plasma flow path in a housing having a blood inlet, a blood cell outlet and plasma outlet and connecting plasma outlets of each separating membrane units with said plasma outlet of the housing, wherein said hydrophilic blood plasma separating membrane comprises a hydrophilic membrane according to any one of claims 1 to 9.

**Patentansprüche**

1. Hydrophile poröse Membran, umfassend:
a) eine poröse Membran mit einem hydrophoben Polymeren $(X)_m$ mit m gleich dem Polymerisationsgrad des hydrophoben Polymeren, bestehend aus mindestens einer Art von Monomereneinheiten X der Formel:

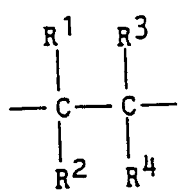

worin bedeuten:
$R^1$ und $R^2$ unabhängig voneinander H oder F;
$R^3$ H, Cl oder F und
$R^4$ H, Cl, F oder $CH_3$, wobei gilt, daß $R^2$ und $R^3$ jeweils für H stehen, wenn nicht $R^1$, $R^2$, $R^3$ und $R^4$ jeweils unveränderlich F bedeuten, und daß $R^3$ für H steht, wenn nicht $R^3$ und $R^4$ jeweils Cl oder F entsprechen, wobei die Monomereneinheiten zur Bildung eines hydrophoben Polymeren fähig sind, als einer Matrix derselben und

b) ein hydrophiles Polymeres $(Y)_n$ mit n gleich dem Polymerisationsgrad des hydrophilen Polymeren, bestehend aus mindestens einer Art von Monomereneinheiten Y der Formel:

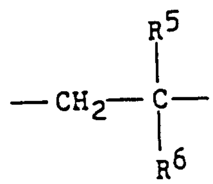

worin $R^5$ für H oder $CH_3$ steht und $R^6$

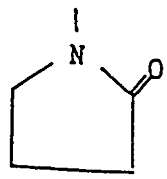

,

$CONH_2$, $CONHR^7$,

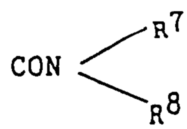

mit $R^7$ und $R^8$ unabhängig gleich Alkyl mit 1 bis 4 Kohlenstoffatom(en),

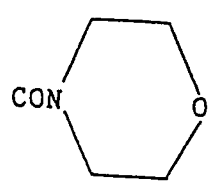

,

COOM mit M gleich einem metallischen Element, $COOR^9NHR^{10}$ mit $R^9$ gleich einem Alkylen mit 1 bis 4 Kohlenstoffatom(en) und $R^{10}$ gleich einem Alkyl mit 1 bis 4 Kohlenstoffatom(en),

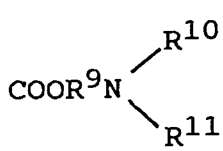

,

worin $R^9$ und $R^{10}$ die zuvor angegebene Bedeutung besitzen und $R^{11}$ ein Alkyl mit 1 bis 4 Kohlenstoffatom(en) darstellt, oder

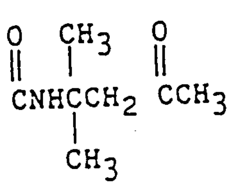

bedeutet, wobei gilt, daß $R^5$ für H steht, wenn $R^6$

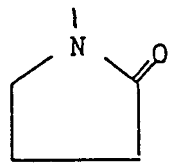

bedeutet,
wobei die Monomereneinheiten Y zur Bildung eines hydrophilen Monomeren fähig sind und wobei die Monomereneinheiten auf mindestens die Oberfläche der porösen Membran pfropfpolymerisiert sind,
und wobei ferner die Monomereneinheiten Y und die Monomereneinheiten X auf der Membranoberfläche in einem Molverhältnis Y/X im Bereich von 0,2 bis 2,0 vorliegen und die in dem gesamten Membrannetzwerk enthaltenen polymeren Komponenten in einem Gewichtsverhältnis $\{Y\}_n/\{X\}_m$ im Bereich von 0,03 bis 0,3 vorhanden sind.

2. Hydrophile poröse Membran nach Anspruch 1, wobei die polymeren Komponenten derart sind, daß in den jeweiligen Formeln der tiefgestellte Index m einen Wert im Bereich von $10^2$ bis $10^5$ und der tiefgestellte Index n einen Wert im Bereich von 10 bis $10^4$ aufweisen.

3. Hydrophile poröse Membran nach Anspruch 1 oder 2, wobei die Monomereneinheiten Y der Formel:

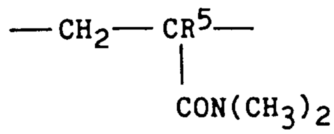

mit $R^5$ gleich H oder $CH_3$ entsprechen.

4. Hydrophile poröse Membran nach einem der Ansprüche 1 bis 3, die einen Blasenpunkt im Bereich von 0,1 bis 5,0 kg/cm$^2$ und eine Wanddicke im Bereich von 50 bis 500 $\mu$m aufweist und unter einem Druck von 0,71 kg/cm$^2$ Wasser in einer Menge von nicht weniger als 5 ml/cm$^2\cdot$min hindurchtreten läßt.

5. Hydrophile poröse Membran nach einem der Ansprüche 1 bis 4, wobei das hydrophobe Polymere aus Polypropylen, einem Ethylen/Propylen-Copolymeren oder Polyvinylidenfluorid besteht.

6. Hydrophile poröse Membran nach einem der Ansprüche 1 bis 5, wobei die hydrophile poröse Membran aus einer hydrophilen Blutplasmatrennmembran besteht.

7. Hydrophile poröse Membran nach Anspruch 6, wobei die Oberfläche der hydrophilen Blutplasmamembran flach ist.

8. Hydrophile poröse Membran nach Anspruch 6, wobei mindestens eine Oberfläche der hydrophilen Blutplasmatrennmembran eine Mehrzahl von sehr kleinen Vorsprüngen aufweist.

9. Hydrophile poröse Membran nach Anspruch 8, wobei auf der die sehr kleinen Vorsprünge aufweisenden Oberfläche eine Pfropfschicht aus dem hydrophilen Polymer gebildet ist.

10. Plasmatrennvorrichtung mit einer Vielzahl von Trennmembraneinheiten mit mindestens einem Plasmaauslaß, die durch Übereinanderlegen zweier hydrophiler Blutplasmatrennmembranen und Abdichten nahe dem Umfangsteil zur Bildung eines Plasmaströmungspfads in einem Gehäuse, das einen Bluteinlaß, einen Blutkörperchenauslaß und einen Plasmaauslaß aufweist und die Plasmaauslässe sämtlicher Trennmembraneinheiten mit dem Plasmaauslaß des Gehäuses verbindet, wobei die hydrophile Blutplasmatrennmembran aus einer hydrophilen Membran nach einem der Ansprüche 1 bis 9

besteht, gebildet sind.

## Revendications

1. Membrane poreuse hydrophile comprenant :

a) une membrane poreuse ayant, en tant que matrice, un polymère hydrophobe $-(X)-_m$, où m est le degré de polymérisation dudit polymère hydrophobe, constitué d'au moins une sorte d'unités monomériques X de formule :

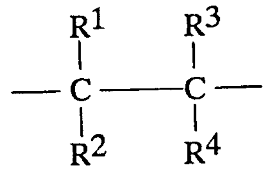

dans laquelle $R^1$ et $R^2$ représentent indépendamment l'un de l'autre H ou F, $R^3$ représente H, Cl ou F, et $R^4$ représente H, Cl, F ou $CH_3$, à condition que $R^2$ et $R^3$ représentent chacun H à moins que $R^1$, $R^2$, $R^3$ et $R^4$ ne représentent chacun de façon invariable F et à condition que $R^3$ représente H à moins que $R^3$ et $R^4$ ne représentent chacun également Cl ou F, lesdites unités monomériques étant capables de former un polymère hydrophobe ; et

b) un polymère hydrophile $-(Y)-_n$, où n est le degré de polymérisation dudit polymère hydrophile, constitué d'au moins une sorte d'unités monomériques Y de formule :

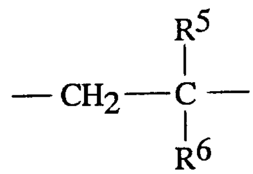

dans laquelle $R^5$ représente H ou $CH_3$ et $R^6$ représente

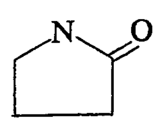 ,

$CONH_2$, $CONHR^7$,

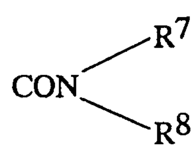

dans lesquels $R^7$ et $R^8$ représentent indépendamment un groupe alkyle en $C_1$-$C_4$,

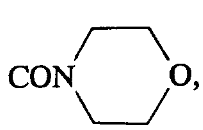 ,

COOM, où M est un élément métallique, $COOR^9NHR^{10}$, où $R^9$ représente un groupe alkylène en

$C_1$-$C_4$ et $R^{10}$ représente un groupe alkyle en $C_1$-$C_4$,

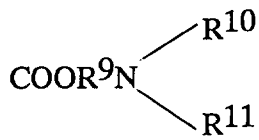

dans lequel $R^9$ et $R^{10}$ ont les mêmes significations que défini ci-dessus et $R^{11}$ représente un groupe alkyle en $C_1$-$C_4$, ou

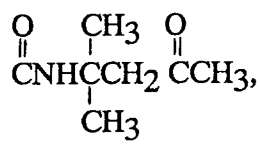

à condition que $R^5$ représente H quand $R^6$ représente

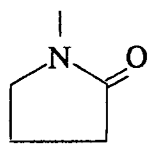 ,

lesdites unités monomériques Y étant capables de former un monomère hydrophile, et étant polymérisées par greffage sur au moins une surface de ladite membrane poreuse;

et lesdites unités monomériques Y et lesdites unités monomériques X étant présentes sur la surface de la membrane dans un rapport molaire X/Y compris dans l'intervalle de 0,2 à 2,0 et lesdits polymères constitutifs étant présents dans tout le tissu de la membrane dans un rapport pondéral $-(Y)-_n/-(X)-_m$ compris dans l'intervalle de 0,03 à 0,3.

**2.** Membrane poreuse hydrophile selon la revendication 1, dans laquelle lesdits polymères constitutifs sont tels que l'indice m a une valeur comprise dans l'intervalle de $10^2$ à $10^5$ et l'indice n a une valeur comprise dans l'intervalle de 10 à $10^4$ dans les formules respectives de ceux-ci.

**3.** Membrane poreuse hydrophile selon la revendication 1 ou 2, dans laquelle lesdites unités monomériques Y sont représentées par la formule

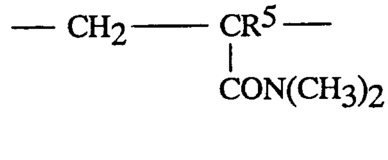

dans laquelle $R^5$ représente H ou $CH_3$.

**4.** Membrane poreuse hydrophile selon l'une quelconque des revendications 1 à 3, dans laquelle ladite membrane poreuse hydrophile possède un point de bulle dans l'intervalle de 0,1 à 5,0 kg/cm² et une épaisseur de paroi dans l'intervalle de 50 à 500 µm et laisse passer l'eau en une quantité supérieure ou égale à 5 ml/cm².min sous une pression de 0,71 kg/cm².

**5.** Membrane poreuse hydrophile selon l'une quelconque des revendications 1 à 4, dans laquelle ledit polymère hydrophobe est du polypropylène, un copolymère d'éthylène et de propylène ou du polyfluorure de vinylidène.

**6.** Membrane poreuse hydrophile selon l'une quelconque des revendications 1 à 5, dans laquelle la membrane poreuse hydrophile est une membrane hydrophile de séparation de plasma sanguin.

**7.** Membrane poreuse hydrophile selon la revendication 6, dans laquelle la surface de ladite membrane hydrophile de séparation de plasma sanguin est plate.

**8.** Membrane poreuse hydrophile selon la revendication 6, dans laquelle au moins l'une des surfaces de ladite membrane hydrophile de séparation de plasma sanguin possède plusieurs protubérances minuscules.

**9.** Membrane poreuse hydrophile selon la revendication 8, dans laquelle une couche greffée dudit polymère hydrophile est formée sur la surface comportant les protubérances minuscules.

**10.** Séparateur de plasma qui comprend l'adaptation de plusieurs unités de membranes de séparation ayant au moins un orifice de sortie de plasma, qui sont formées en superposant deux membranes hydrophiles de séparation de plasma sanguin et en les scellant à proximité de la circonférence afin de former un passage pour le courant de plasma dans un boîtier comportant un orifice d'admission du sang, un orifice de sortie des cellules sanguines et un orifice de sortie du plasma et la connexion des orifices de sortie du plasma de chaque unité de membranes de séparation avec ledit orifice de sortie du plasma du boîtier, dans lequel ladite membrane hydrophile de séparation du plasma sanguin comprend une membrane hydrophile selon l'une quelconque des revendications 1 à 9.

FIG.1

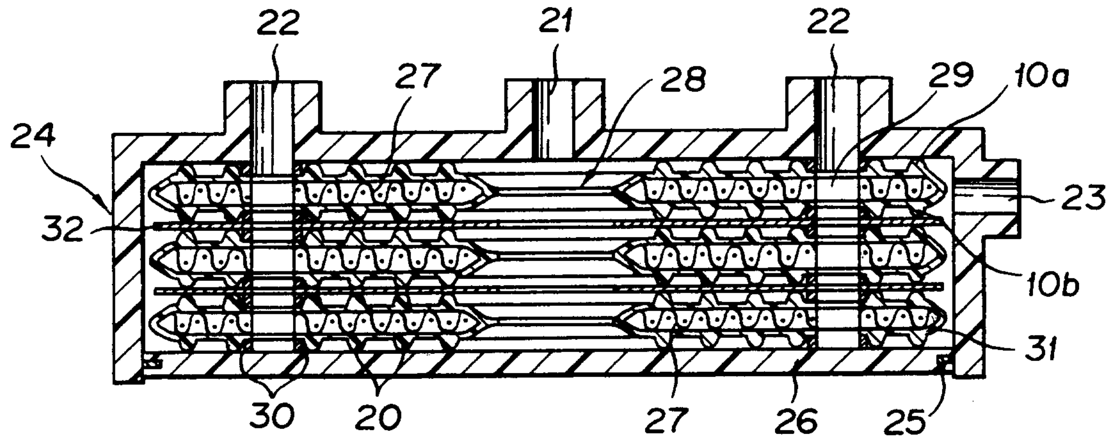
22
21
22
27
28
29
10a
24
23
32
10b
31
30
20
27
26
25

FIG.2

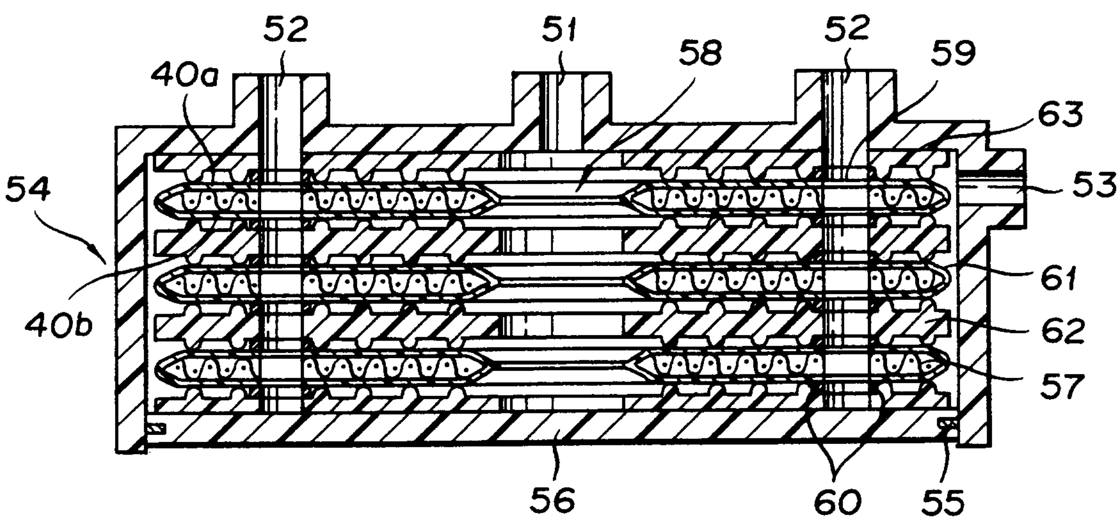
52
51
52
40a
58
59
63
54
53
61
40b
62
57
56
60
55